# EUROPÄISCHE PATENTSCHRIFT

(11) **EP 1 362 879 B1**
(45) Veröffentlichungstag und Bekanntmachung des Hinweises auf die Patenterteilung: **02.04.2008**
(21) Anmeldenummer: 03008058.4
(22) Anmeldetag: 14.04.2003
(51) Int. Cl.: C08G 81/00, C08L 67/07, C08L 75/06

(54) **Interpenetrierende Netzwerke**
Interpenetrating networks
Résaux interpénétrants

(30) Priorität: 18.04.2002 DE 10217351
(43) Veröffentlichungstag der Anmeldung: 19.11.2003
(73) Patentinhaber: Mnemoscience GmbH, 52074 Aachen (DE)
(72) Erfinder: Lendlein, Andreas, 14167 Berlin (DE); Schmidt, Annette, 41462 Neuss (DE)
(74) Vertreter: Grünecker, Kinkeldey, Stockmair & Schwanhäusser Anwaltssozietät

(56) Entgegenhaltungen:
- WO-A-01/91822
- WO-A-99/42147
- WO-A-99/42528
- US-A- 6 160 084
- ANNETTE M. SCHMIDT: "Bioabbaubare Polymernetzwerk-Systeme mit Formgedächtniseffekt und kristallisierbarem Schaltsegment" 6. Juni 2002 (2002-06-06) , MENSCH UND BUCH VERLAG , BERLIN XP002260848 * Seite I-X * * Seite 135-175 *

## Beschreibung

Die vorliegende Erfindung betrifft interpenetrierende Netzwerke aus einer kovalent vemetzten Polymerkomponente und einer Polyesterurethankomponente.

Polymere Werkstoffe sind wichtige Bausteine in vielen Anwendungsbereichen, in denen die klassischen Werkstoffe, wie Metalle, Keramik und Holz, aufgrund ihrer beschränkten physikalischen Eigenschaften nicht mehr ausreichend sind. Polymere Werkstoffe haben sich daher ein breites Anwendungsgebiet erobert, nicht zuletzt auch dadurch, dass sich durch Variation der monomeren Bausteine der polymeren Werkstoffe die Werkstoffeigenschaften variieren lassen. Eine insbesonders faszinierende Klasse an polymeren Werkstoffen, die in den vergangenen Jahren entwickelt wurden, sind die sogenannten Form-Gedächtnis-Polymere (im folgenden auch Shape Memory Polymere, SMP oder SMP-Materialien genannt), d.h. polymere Werkstoffe, die eine oder sogar mehrere Formen m "Gedächtnis" behalten können, wodurch gezielte Formveränderungen durch äußere Reize, wie Temperaturveränderung, ausgelöst werden können. Solche Materialien sind beispielsweise in den internationalen Patentanmeldungen WO-A-99-42528 und WO-A-99-42147 beschrieben. Ein Nachteil der dort z.B. beschriebenen thermoplastischen Materialien ist aber, dass beim wiederholten Durchlaufen eines Zyklus von Formveränderungen häufig nicht die erwünschte genaue Wiederherstellung der Ausgangsform erreicht wird. Darüber hinaus neigen diese Materialien des Stands der Technik aufgrund von irreversiblen Kriechprozessen bei wiederholtem Verformen häufig zum "Ausleiern", so dass erwünschte physikalische Eigenschaften im Verlauf von einigen Zyklen verloren gehen. Die US 6,160,084 A offenbart Form-Gedächtnis-Polymere.

Es ist daher die Aufgabe der vorliegenden Erfindung polymere Werkstoffe anzugeben, die die Nachteile des Stands der Technik überwinden. Die polymeren Werkstoffe sollten darüber hinaus die Möglichkeit eröffnen, dass durch einfache Variation der Zusammensetzung eine Eigenschaftensteuerung möglich wird, wodurch gezielt Materialien mit einem erwünschten Werkstoffprofil erhalten werden können.

Die vorliegende Erfindung löst diese Aufgabe durch das interpenetrierende Netzwerk nach Anspruch 1. Bevorzugte Ausführungsformen sind in den Unteransprüchen angegeben. Darüber hinaus stellt die vorliegende Erfindung ein Verfahren zur Herstellung des erfindungsgemäßen interpenetrierenden Netzwerks zur Verfügung, wie in Anspruch 7 definiert. Bevorzugte Ausführungsformen sind wiederum in den Unteransprüchen angegeben.

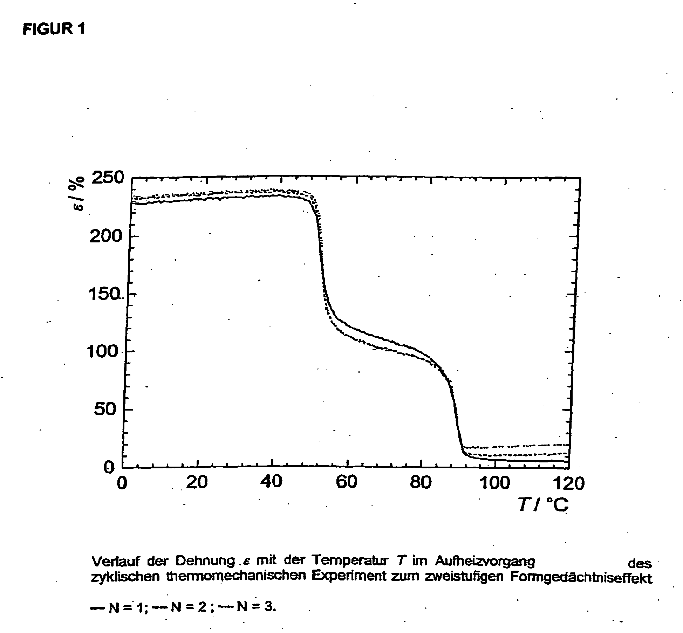

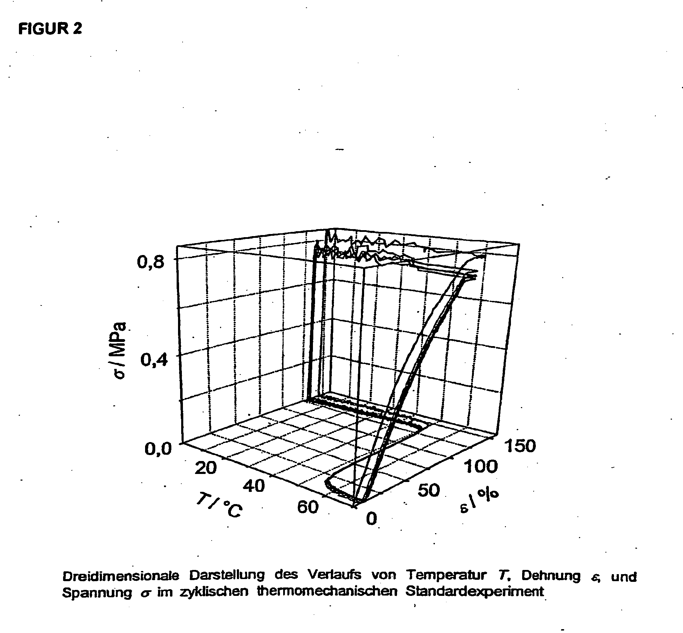

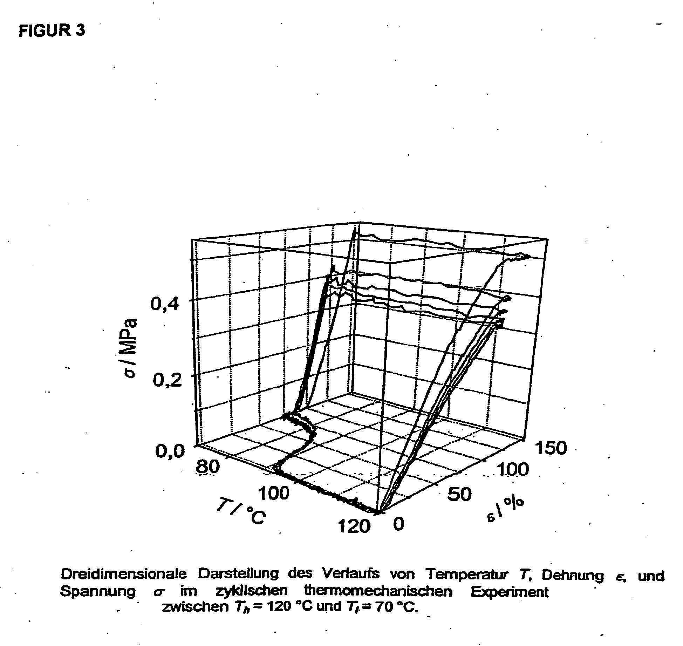

Figur 1 zeigt Zug-Dehnungs-Messungen mit einem erfindungsgemäßen Netzwerk (Versuch 9). Figuren 2 und 3 zeigen jeweils dreidimensionale Darstellungen von Zug-Dehnungs-Messungen unter Berücksichtigung der Temperatur für erfindungsgemäße Materialien (Versuch 8 bzw. Versuch 9).

Im folgenden wird die vorliegende Erfindung detailliert beschrieben.

Die interpenetrierenden Netzwerke im Sinne der Erfindung umfassen kovalent vernetzte Polymere, die von einer getrennt davon vorliegenden weiteren polymeren Komponente durchdrungen sind. Diese weitere polymere Komponente lässt sich durch physikalische Methoden nicht von dem Netzwerk abtrennen, ist selbst allerdings nicht vernetzt, weder mit Molekülen der eigenen Art noch mit der vernetzten Komponente. Die beiden wesentlichen polymeren Komponenten des erfindungsgemäßen Netzwerks werden im folgenden erläutert.

### 1. Kovalent vernetzte Komponente

Das erfindungsgemäße Netzwerk umfasst eine Polymerkomponente, die nicht lediglich physikalische Interaktionen zeigt sondern kovalent vernetzt vorliegt.

Diese Komponente wird bevorzugt erhalten durch Vernetzen von funktionalisierten Makromonomeren. Die Funktionalisierung erlaubt bevorzugt eine kovalente Verknüpfung der Makromonomere durch Reaktionen, die keine Nebenprodukte ergeben. Bevorzugt wird diese Funktionalisierung durch ethylenisch ungesättigte Einheiten zur Verfügung gestellt, insbesondere bevorzugt durch Acrylatgruppen und Methacrylatgruppen, wobei letztere insbesondere bevorzugt sind. Die Makromonomere sind bevorzugt Polyestermakromonomere, insbesondere bevorzugt Polyestermakromonomere auf der Basis von Caprolacton. Andere mögliche Polyestermakromonomere basieren auf Lactideinheiten, Glycolideinheiten, p-Dioxanoneinheiten und deren Mischungen und Mischungen mit Caprolactoneinheiten, wobei Polyestermakromonomere mit Caprolactoneinheiten insbesondere bevorzugt sind.

Werden die oben beschriebenen Makromonomere vernetzt, so entstehen Netzwerke mit einer einheitlichen Struktur, wenn lediglich eine Art an Makromonomer eingesetzt wird. Werden zwei Arten an Makromonomeren eingesetzt, so werden Netzwerke vom AB-Typ erhalten. Solche Netzwerke vom AB-Typ können auch erhalten werden, wenn die funktionalisierten Makromonomere mit geeigneten niedermeolekularen oder oligomeren Verbindungen copolymerisiert werden. Sind die Makromonomere mit Acrylatgruppen oder Methacrylatgruppen funktionalisiert, so sind geeignete Verbindungen, die copolymersisiert werden können, niedermolekulare Acrylate, Metharylate, Diacrylate oder Dimethacrylate. Bevorzugte Verbindungen dieser Art sind Acrylate, wie Butylacrylat oder Hexylacrylat, und Methacrylate, wie Methylmethacrylat und Hydroxyethylmethacrylat.

Diese Verbindungen, die mit den Makromonomeren copolymerisiert werden können, können in einer Menge von 5 bis 70 Gew.-%, bezogen auf das Netzwerk aus Makromonomer und der niedermolekularen Verbindung vorliegen, bevorzugt in einer Menge von 15 bis 60 Gew.-%. Der Einbau von variierenden Mengen der niedermolekularen Verbindung erfolgt durch Zugabe entsprechender Mengen an Verbindung zur zu vernetzenden Mischung. Der Einbau der niedermolekularen Verbindung in das erfindungsgemäße Netzwerk erfolgt in einer Menge, die der in der Vernetzungsmischung enthaltenen Menge entspricht.

Die erfindungsgemäß zu verwendenden Makromonomere werden im folgenden detailliert beschrieben.

Die kovalent zu vernetzenden Makromonomere weisen bevorzugt ein Zahlenmittel des Molgewichts, bestimmt durch GPC-Analyse von 2000 bis 30000 g/mol, bevorzugt von 5000 bis 20000 g/mol und insbesondere bevorzugt von 7500 bis 15000 g/mol auf. Die kovalent zu vernetzenden Makromonomere weisen bevorzugt an beiden Enden der Makromonomerkette eine Methacrylatgruppe auf. Eine derartige Funktionalisierung erlaubt die Vernetzung der Makromonomere durch einfache Photoinitiation (Bestrahlung).

Insbesondere bevorzugt sind die erfindungsgemäß einzusetzenden Makromonomere Polyester, umfassend die vernetzbaren Endgruppen. Ein insbesondere bevorzugter, erfindungsgemäß einzusetzenden Polyester ist ein Polyester auf der Basis von Caprolacton, für den die oben aufgeführten Angaben über das Molgewicht gelten. Die Herstellung eines solchen Polyestermakromonomeren, an den Enden funktionalisiert, bevorzugt mit Methacrylatgruppen, kann durch einfache Synthesen, die dem Fachmann bekannt sind hergestellt werden. Diese Netzwerke, ohne Berücksichtigung der weiteren wesentlichen polymeren Komponente der vorliegenden Erfindung, zeigen semikristalline Eigenschaften und weisen einen Schmelzpunkt der Polyesterkomponente auf (bestimmbar durch DSC-Messungen), der abhängig von der Art der eingesetzten Polyesterkomponente ist und darüber somit auch steuerbar ist. Im allgemeinen liegt diese Temperatur (Tm1) jedoch in der Nähe von 50°C.

### 2. Nicht-kovalent vernetzte Komponente

Das erfindungsgemäße interpenetrierende Netzwerk umfasst weiter eine Komponente aus Polyesterurethanen wie in Anspruch 1 definiert. Diese Polyesterurethane liegen im erfindungsgemäßen Netzwerk nicht kovalent vernetzt vor, sondern es liegen zwischen den verschiedenen Bereichen der Polyesterurethane allenfalls physikalische Wechselwirkungen vor.

Die im erfindungsgemäßen interpenetrierenden Netzwerk vorliegenden Polyesterurethane umfassen bevorzugt als Esterkomponente Einheiten, abgeleitet von Caprolacton und Pentadecalacton. Weitere mögliche Esterkomponenten sind Einheiten auf der Basis von p-Dioxanon und anderen Estersegmente formenden Verbindungen, die für Form-Gedächtnis-Materialien bekannt sind und schon oben im Zusammenhang mit den Makromonomeren aufgelistet wurden. Bevorzugt sind jedoch in dieser Erfindung die Polyesterurethane die als Esterkomponente Einheiten, abgeleitet von Caprolacton und Pentadecalacton aufweisen.

Die Esterkomponente in den Polyesterurethanen hat bevorzugt ein Zahlenmittel des Molgewicht von 1000 bis 20000, insbesondere bevorzugt von 1500 bis 15000 g/mol, betimmt durch GPC. Die Esterkomponente kann in Form von Homopolyesterblöcken oder Copolyesterblöcken vorliegen, bevorzugt sind Homopolyesterblöcke. Liegen Caprolactoneinheiten und Pentadecalactoneinheiten gemeinsam im erfindungsgemäß eingesetzten Polyesterurethan vor, so ist es bevorzugt, in Übereinstimmung mit dem oben Gesagten, wenn die Caprolactoneinheiten bzw. die Pentadecalactoneinheiten jeweils als Homopolyesterblöcke (im folgenden auch Segmente genannt) im Polyesterurethan vorliegen.

In bevorzugten Ausführungsformen werden Polypentadecalactonsegmente in Polyesterurethanen eingesetzt. Bei bevorzugten Ausführungsformen der vorliegenden Erfindung werden die Polypentadecalactonsegmente als Hartsegment in Polyesterurethanen eingesetzt, die neben den Polypentadecalactonsegmenten noch andere Polyestersegmente, bevorzugt Polycaprolactonsegmente, als Weichsegmente enthalten.

Das Polypentadecalactonsegment, enthalten im erfindungsgemäß eingesetzten Polyesterurethan wird üblicherweise in Form eines Makrodiols in das Polyesterurethan eingeführt. Dieses Segment kann durch ringöffnende Polymerisation aus ω-Pentadecalacton unter Zinnkatalyse und Einsatz von Ethylenglycol als Initiator erhalten werden. Durch das Verhältnis von Initiator zu Monomer kann das Molgewicht des Segments eingestellt werden. Das Molgewicht der Polypentadecalactonsegmente im erfindungsgemäßen Polyesterurethan ist nicht kritisch. Üblicherweise beträgt das Zahlenmittel des Molgewichts jedoch 1000 bis 20000 g/mol, bevorzugt 2000 bis 11000 g/mol, bestimmt durch GPC-Analyse. Das Makrodiol aus Pentadecalacton kann mit den bei der Herstellung von Polyurethanen üblichen Diisocyanaten zu Polyesterurethanen umgesetzt werden. Bevorzugte Diisocyanate sind dabei Verbindungen der Formel O=C=N-R-N=C=O, wobei R aromatisch oder aliphatisch sein kann. Bevorzugt ist R jedoch aliphatisch, mit einer Kohlenstoffkette mit 1 bis 10, bevorzugt 2 bis 8, insbesondere bevorzugt 4 bis 7 Kohlenstoffatomen. Diese Kohlenstoffkette kann mit Wasserstoff abgesättigt sein oder weitere Substituenten aufweisen. Diese Substituenten umfassen kurzkettige Alkylgruppen, insbesondere Methylgruppen. Ein insbesondere bevorzugtes Diisocyanat ist ein Trimethylhexan-1,6-diisocyanat.

Durch die Variation des Molgewichts des Polypentadecalactonsegments lassen sich die Eigenschaften des Polyesterurethans variieren. Das Molgewicht des Polyesterurethans ist nicht kritisch und kann in Abhängigkeit vom erwünschten Verwendungszweck gewählt werden. Typische Molgewichte (Zahlenmittel, bestimmt durch GPC) sind im Bereich von 100000 und 250000 g/mol, bevorzugt im Bereich von 150000 bis 200000 g/mol.

Die oben gemachten Ausführungen gelten auch für Polyesterurethane die als Esterkomponente Polycaprolactonsegmente umfassen.

Bevorzugt enthält das Polyesterurethan, wenn ein Polypentadecalactonsegment vorliegt, noch mindestens ein weiteres Segment, wobei dieses zusätzliche Segment unter einer Vielzahl an chemisch verschiedenen Komponenten gewählt werden kann, wie teilkristalline Segmente, umfassend Polyestersegmente, Polyetherestersegmente und Polyethersegmente wie Polycaprolactonsegmente (PCL), Polycaprolacton-copolytetrahydrofuransegmente (PCL-co-pTHF), Polytetrahydrofuransegmente (pTHF), Polypropylenglycolsegmente (PPG) und Polyethylenglycolsegmente (PEG), sowie glasartige Segmente, umfassend Polyester und Copolyester, wie Poly-L-lactid-co-glycolid (ran) (PLGA) und Poly-DL-Lactid (P-DL-LA), insbesondere bevorzugt ein Polycaprolactonsegment. Diese Polyesterurethane sind Blockcopolymere mit Polypentadecalactonsegmenten, verknüpft mit anderen Segmenten, bevorzugt Polycaprolactonsegmenten. Die anderen Segmente, bevorzugt das Polycaprolactonsegment können, wie oben für das Polypentadecalactonsegment beschrieben, in Form eines Makrodiols in das erfindungsgemäße Polyesterurethan eingeführt werden. Dieses Makrodiol kann z.B. durch ringöffnende Polymerisation von s-Caprolacton, analog dem oben beschriebenen Verfahren erhalten werden.

Das Molgewicht der weiteren Segmente, wie des Polycaprolactonsegments ist nicht kritisch, üblicherweise weisen diese Segmente jedoch ein Zahlenmittel des Molgewichts, bestimmt durch GPC, von 1000 bis 20000 g/mol auf, bevorzugt von 2000 bis 11000 g/mol, wobei der bevorzugte Bereich für PEG-Segmente von 2000 bis 20000 g/mol, für PLGA von 4000 bis 9000 g/mol und für P-DL-LA von 5000 bis 11000 g/mol ist. Die Polyesterurethane mit den weiteren Segmenten, bevorzugt Polycaprolactonsegmenten, weisen bevorzugt ein Molgewicht von 50000 bis 250000 g/mol (Zahlenmittel, bestimmt durch GPC) auf, stärker bevorzugt von 60000 bis 200000 g/mol, insbesondere bevorzugt 62000 bis 196000 g/mol (und in einigen Ausführungsformen auch von 55000 bis 100000 g/mol). Der Anteil an Pentadecalactoneinheiten kann über einen breiten Bereich variieren, bevorzugt liegt der Anteil an Pentadecalactoneinheiten im Bereich von 10 bis 80 Gew.-%, insbesondere bevorzugt im Bereich von 20 bis 60 Gew.-%.

Für die besonders bevorzugte Ausführungsform gilt, dass das Molgewicht des Polycaprolactonsegments nicht kritisch ist. Üblicherweise weist dieses Segment jedoch ein Zahlenmittel des Molgewichts, bestimmt durch GPC, von 1000 bis 20000 g/mol auf, bevorzugt von 2000 bis 11000 g/mol. Die Polyesterurethane mit Polycaprolactonsegmenten weisen bevorzugt ein Molgewicht von 100000 bis 250000 g/mol (Zahlenmittel, bestimmt durch GPC) auf, stärker bevorzugt von 120000 bis 190000 g/mol. Der Anteil an Pentadecalactoneinheiten kann über einen breiten Bereich variieren, bevorzugt liegt der Anteil an Pentadecalactoneinheiten im Bereich von 10 bis 80 Gew.-%, insbesondere bevorzugt im Bereich von 20 bis 60 Gew.-%.

Gute interpenetrierende Netzwerke der vorliegenden Erfindung lassen sich mit den folgenden Polyesterurethanen herstellen:
Polypentadecalactonsegment: Molgewicht 1000 bis 10000 g/mol (Zahlenmittel), bevorzugt 1500 bis 5000, insbesondere bevorzugt 2000 bis 3000 g/mol
Polycaprolactonsegment: Molgewicht 3000 bis 11000 g/mol (Zahlenmittel), bevorzugt 4000 bis 10000 g/mol
Polycaprolacton-co-polytetrahydrofuransegment: Molgewicht 1000 bis 5000 g/mol (Zahlenmittel), bevorzugt 1500 bis 3500 g/mol
Polytetrahydrofuransegment: Molgewicht 1000 bis 5000 g/mol (Zahlenmittel), bevorzugt 1500 bis 3000 g/mol
Polypropylenglycolsegment: Molgewicht 1000 bis 8000 g/mol (Zahlenmittel), bevorzugt 1200 bis 4500 g/mol
Polyethylenglycolsegment: Molgewicht 1000 bis 25000 g/mol (Zahlenmittel), bevorzugt 1500 bis 20000 g/mol
Poly-L-lactid-co-glycolidsegmnet (ran): Molgewicht 4000 bis 10000 g/mol (Zahlenmittel), bevorzugt 5000 bis 8000 g/mol
Poly-DL-lactidsegment: Molgewicht 4000 bis 15000 g/mol (Zahlenmittel), bevorzugt 5000 bis 11000 g/mol
Polyesterurethan: Molgewicht 50000 bis 200000 g/mol (Zahlenmittel), bevorzugt 60000 bis 190000 g/mol; Anteil weiteres Segment 20 bis 80 Gew.-%, bevorzugt 45 bis 70 Gew.-%, weiter bevorzugt 50 bis 60 Gew.-%, Anteil Polypentadecalactonsegment 80 bis 20 Gew.-%, bevorzugt 55 bis 30 Gew.-%, weiter bevorzugt 40 bis 50 Gew.-%

Werden die beiden oben beschriebenen Polyestersegmente durch Polyaddition mit den oben beschriebenen Diisocyanaten zu erfindungsgemäßen Polyesterurethanen umgesetzt, so lässt sich durch Variation der jeweiligen Anteile und Molmassen der Polyestersegmente das Eigenschaftsprofil der resultierenden Polyesterurethane über einen breiten Bereich einstellen. In dieser bevorzugten Ausführungsform der vorliegenden Erfindung wird also ein polymeres System zur Verfügung gestellt, das durch Variation einfacher Ausgangsmaterialien eine gezielte Eigenschaftenmodifizierung ermöglicht. Die erfindungsgemäßen Netzwerke weisen durch die Modifikationsmöglichkeiten des einzusetzenden Polyesterurethans eine Reihe von Variablen zur Eigenschaftsmodifizierung auf, wie Länge der Segmente im Polyesterurethan, Gehalt an Polyesterurethan im Netzwerk und chemische Zusammensetzung des Polyesterurethans.

Die bevorzugten Polyesterurethane der vorliegenden Erfindung, die neben den Polypentadecalactonsegmenten noch Polycaprolatonsegmente aufweisen, zeichnen sich durch weitere bevorzugte Eigenschaftsprofile aus.

Die Verwendung von Polyesterurethanen mit Polycaprolactonsegmenten hat für die erfindungsgemäßen Netzwerke den Vorteil, dass identische Segmente im kovalent vernetzten Teil und im Polyesterurethan vorliegen (wenn auch das Netzwerk Caprolactoneinheiten umfasst), die eine gemeinsame kristalline Phase bilden können. Durch diese Cokristallisation wird die Durchdringung im interpenetrierenden Netzwerk der vorliegenden Erfindung auf molekularer Ebene gewährleistet und verbessert, so dass auch bei der Herstellung der erfindungsgemäßen bevorzugten interpenetrierenden Netzwerke weniger Probleme auftreten können.

Der Einsatz von Polyesterurethanen mit Polypentadecalactonsegmenten hat für die erfindungsgemäßen interpenetrierenden Netzwerke weitere Vorteile. In erfindungsgemäßen Materialien, die beispielsweise Polycaprolactonsegmente aufweisen, mit einer Schmelztemperatur bei 50°C (siehe oben), kann durch die Einführung der Polypentadecalactonsegmente eine zweite Schmelztemperatur (bestimmbar durch DSC-Messungen) eingeführt werden. Diese zweite Temperatur (Tm2) liegt in der Nähe von 90°C. Die Polycaprolactonsegmente und die Polypentadecalactonsegmente bilden drüber hinaus keine Mischkristalle sondern jeweils eigenen Phasen.

Weiterhin können die mechanischen Eigenschaften gezielt über einen weiten Bereich variiert werden. Mit steigendem Anteil an Polypentadeclacton kann der Wert für das E-Modul des Polyesterurethans gesteigert werden. Der Wert für die Bruchdehnung kann in einem Bereich von 600 bis 1200% mit steigendem Polypentadecalactongehalt eingestellt werden und auch die Zugfestigkeit lässt sich mit steigendem Gehalt an Polypentadecalactonsegment über einen Bereich von 4 bis 10 MPa einstellen (alle Werte bestimmt bei 70°C). Durch die geringere, d.h. langsamere Bioabbaubarkeit der Polypentadecalactonsegmente, verglichen mit z.B. Polyparadioxanonsegmenten, können auch die bevorzugten Polyesterurethane der vorliegenden Erfindung in Anwendungen zum Einsatz kommen, in denen die Polyesterurethane mit Polyparadioxanonsegmenten aufgrund der schnellen Abbaubarkeit und der damit verbundenen ungenügenden mechanischen Stabilität nicht einsetzbar sind. Im Vergleich mit den Polyesterurethanen mit Polycaprolactonsegmenten und Polyparadioxanonsegmenten zeichnen sich die bevorzugten Polyesterurethane darüber hinaus durch eine vergrößerte Produktionsstabilität und Granulierfähigkeit aus, was die Herstellung und Handhabung der erfindungsgemäßen Netzwerke vereinfacht.

In den erfindungsgemäßen Netzwerken, so haben Untersuchungen gezeigt, führt eine Erhöhung des Anteils an Polyesterurethan zu einer Erhöhung der Bruchdehnung, bei 22°C z.B. von 250 % auf 450%. Gleichzeitig steigt die Differenz zwischen Streckspannung und Zugfestigkeit.

Insbesondere bevorzugte Polyesterurethane, verwendet in der vorliegenden Erfindung, die sowohl Polypentadecalactonsegmente als auch Polycaprolactonsegemente aufweisen zeigen darüber hinaus Form-Gedächnis-Eigenschaften, so dass diese bevorzugten Materialien schon selbst als Shape-Memory-Polymere (SMP) bezeichnet werden können.

Die erfindungsgemäßen, interpenetrierenden Netzwerke werden erhalten durch das Vernetzen der endgruppenfunktionalisierten Makromonomere in der Gegenwart der Polyesterurethane. Zusätzlich zu den Makromonomeren können, wie bereits vorstehend ausgeführt, niedermolekulare Comonomere, wie Acrylate oder Methacrylate, beispielsweise Alkylacrylate oder Alkylmethacrylate eingesetzt werden, so dass kovalent vernetzte AB-Netzwerke entstehen. Diese Vernetzung kann erreicht werden durch das Bestrahlen einer Mischung, umfassend die Polyesterurethankomponente und die endgruppenfunktionalisierte Makromonomerkomponnente und ggf. ein niedermolekulares Comonomer. Geeignete Verfahrensbedingungen dafür sind das Bestrahlen der Mischung in Schmelze, mit Licht einer Wellenlänge von vorzugsweise 308 nm. Die zu vernetzende Mischung wird vorzugsweise vor dem Aufschmelzen und Vernetzen erhalten durch Lösen der einzusetzenden Ausgangskomponenten in einem geeigneten Lösungsmittel und Ausfällen der Mischung aus der Lösung in einem Fällmittel. Bevorzugte Lösungsmittel sind inerte, polare Lösungsmittel, insbesondere Chloroform. Die herzustellende Lösung weist bevorzugt eine Feststoffkonzentration von 2 bis 20 Gew.-% auf, insbesondere bevorzugt 8 bis 12 Gew.-%. Die Ausfällung erfolgt bevorzugt durch Eintropfen der Lösung in ein geeignetes inertes, unpolares Fällmittel, bevorzugt ein aliphatisches Kohlenwasserstofffällmittel, insbesondere bevorzugt die Hexanfraktion. Die Ausfällung erfolgt quantitativ, so dass bei der Lösungsherstellung die gewünschten Mischungsverhältnisse an Polyesterurethankomponete und funktionalisierter Makromonomerkomponente eingestellt werden können. Vor der Umsetzung in der Schmelze wird die ausgefällte Mischung bevorzugt noch getrocknet, insbesondere bevorzugt bei vergleichsweise milden Bedingungen, wie 25 bis 40 °C und Umgebungsdruck.

Die Vernetzung, die wie oben beschrieben erfolgen kann, ergibt ein interpenetrierendes Netzwerk aus kovalent verknüpfter Makromonomerkomponente, darin verteilt die nicht kovalent verknüpften Polyesterurethane. Diese Polyesterurethane können jedoch physikalisch interagierende Bereiche ausbilden, die durch die gegebenenfalls kristallin vorliegenden Polyestersegmente der Polyesterurethane gebildet werden.

Die erfindungsgemäßen interpenetrierenden Netzwerke können variierende Mengen der einzelnen Bestandteile aufweisen.

Geeignete Mengen an endgruppenfunktionalisierter Makromonomerkomponente liegen zwischen 5 und 99 Gew.-%, bezogen auf die Mischung aus Makromonomerkomponente und Polyesterurethankomponente, bevorzugt zwischen 40 und 95 Gew.-% und insbesondere bevorzugt zwischen 60 und 90 Gew.-%.

Die Polyesterurethankomponente liegt somit im Allgemeinen in einer Menge von 1 bis 95 Gew.-% vor, bevorzugt von 5 bis 60 Gew.-%, insbesondere bevorzugt von 10 bis 40 Gew.-%, bezogen auf die Mischung aus Makromonomerkomponente und Polyesterurethankomponente.

Die Polyesterurethankomponete umfasst bevorzugt Polyesterurethane auf Basis von Caprolacton und Pentadecalacton. Die Gesamtmenge, im interpenetrierenden Netzwerk der Erfindung, an Pentadecalacton liegt bevorzugt im Bereich von 2 bis 30 Gew.-%, insbesondere bevorzugt im Bereich von 6 bis 25 Gew.-%, bezogen auf die Mischung aus Makromonomerkomponente und Polyesterurethankomponente.

Die interpenetrierenden Netzwerke der vorliegenden Erfindung zeichnen sich durch die folgenden Eigenschaften aus.

Insgesamt sind die interpenetrierenden Netzwerke der vorliegenden Erfindung gute SMP-Materialien, mit hohen Rückstellwerten, d.h. die ursprüngliche Form wird auch bei mehrfachem Durchlaufen eines Zyklus an Formänderungen zu einem hohen Prozentsatz, üblicherweise oberhalb von 90%, erneut erhalten. Dabei tritt auch kein nachteiliger Verlust an mechanischen Eigenschaftswerten auf. Die Netzwerke mit Polyesterurethanen auf Basis von Caprolacton und Pentadecalacton zeigen zwei Schmelzpunkte (Umwandlungsgspunkte) assoziert mit zwei Formveränderungspunkten, so dass diese bevorzugten erfindungsgemäßen Materialien zwei unterschiedliche Formen im "Gedächtnis" behalten können. Die Form-Gedächtnis-Eigenschaften der Materialien der vorliegenden Erfindung werden nachfolgend kurz definiert.

Form-Gedächtnis-Polymere im Sinne der vorliegenden Erfindung sind Materialien, die durch ihre chemisch-physikalische Struktur in der Lage sind, gezielte Formänderungen durchzuführen. Die Materialien besitzen neben ihrer eigentlichen permanenten Form eine weitere Form, die dem Material temporär aufgeprägt werden kann. Solche Materialien sind durch zwei Merkmale charakterisiert. Sie umfassen sogenannte Schaltsegmente, die einen extern stimulierten Übergang auslösen können, üblicherweise durch eine Temperaturänderung. Darüber hinaus umfassen diese Materialien kovalente Vernetzungspunkte, die für die sogenannte permanente Form verantwortlich sind. Diese permanente Form wird durch die dreidimensionale Struktur eines Netzwerks gekennzeichnet. Die in der vorliegenden Erfindung im erfindungsgemäßen Netzwerk vorliegenden Vernetzungspunkte sind kovalenter Natur und werden in den bevorzugten Ausführungsformen der vorliegenden Erfindung erhalten durch die Polymerisation der Methacrylatendgruppen. Die Schaltsegmente, die den thermisch induzierten Übergang (Formveränderung) auslösen, sind in der vorliegenden Erfindung, bezogen auf die bevorzugten Ausführungsformen, die Polycaprolactonsegmente bzw. Polypentadecalactonsegmente, die durch Änderung der kristallinen bzw. nichtkristallinen Struktur eine Formveränderung initiieren. Der thermische Übergangspunkt wird definiert durch die Schmelztemperaturen der kristallinen Bereiche (Tm). Oberhalb von Tm befindet sich das Material im amorphen Zustand und ist elastisch. Wird also eine Probe über die Übergangstemperatur Tm erwärmt, im flexiblen Zustand dann deformiert und wieder unter die Übergangstemperatur abgekühlt, so werden die Kettensegmente durch Einfrieren von Freiheitsgraden im deformierten Zustand fixiert (Programmierung). Es werden temporäre Vernetzungsstellen (nichtkovalent) geformt, so dass die Probe auch ohne äußere Last nicht mehr in ihre ursprüngliche Form zurück kehren kann. Beim erneuten Erwärmen auf eine Temperatur oberhalb der Übergangstemperatur werden diese temporären Vernetzungsstellen wieder aufgelöst und die Probe kehrt zu ihrer ursprünglichen Form zurück. Durch erneutes Programmieren kann die temporäre Form wieder hergestellt werden. Die Genauigkeit, mit der die ursprüngliche Form wieder erhalten wird, wird als Rückstellverhältnis bezeichnet. Bei interpenetrierenden Netzwerken der Erfindung die zwei Übergangstemperaturen (Tm) aufweisen, d.h. den bevorzugten Systemen in denen Caprolactonsegmente und Pentadecalactonsegmente vorliegen, können in der Art und Weise wie oben geschildert nacheinander zwei temporäre Formen programmiert werden. Die erste, permanente Form wird dabei wie gehabt durch die kovalenten Vernetzungsstellen fixiert. Die zweite temporäre Form wird durch Verformung des Materials oberhalb der oberen Übergangstemperatur Tm2 und Abkühlen einprogrammiert. Für die Fixierung dieser Form sind kristalline Vernetzungspunkte der Pentadecalactonsegmente verantwortlich. Die dritte, wiederum temporäre Form wird bestimmt durch die untere Übergangstemperatur Tm1 (Programmierung durch Verformung oberhalb dieser Temperatur und Abkühlen). Für die Fixierung dieser Form sind die kristallinen Vernetzungspunkte der Caprolactonsegmente verantwortlich. Durch geeignete Zug-Dehnungsexperimente kann der Form-Gedächtnis-Effekt gezeigt werden. Ein Beispiel solchen Zug-Dehnungs-Messungen ist in Figur 1 gezeigt. Das dort untersuchte Material, ein interpenetrierendes Netzwerk mit kovalent vernetzten Polycaprolactonsegmenten und eine Polyesterurethankomponente auf der Basis von Caprolacton und Pentadecalacton, zeigt zwei abrufbare Formänderungen, angezeigt durch die beiden Stufen im Diagramm. Die Tatsache, dass die drei Wiederholungen der Messung sehr ähnliche Ergebnisse zeigen (kaum Abweichung der Meßdaten) zeigt an, dass das Material auch ein sehr gutes Rückstellverhältnis aufweist, sowie eine gute Beibehaltung des Form-Gedächtnis-Effekts.

Die erfindungsgemäßen interpenetrierenden Netzwerke der vorliegenden Erfindung können, neben den oben diskutierten wesentlichen Komponenten weitere Stoffe enthalten, solange die Funktion der Netzwerke nicht beeinträchtigt wird. Solche zusätzlichen Materialien können beispielsweise Färbmittel, Füllstoffe oder zusätzliche polymere Materialien sein, die für verschiedene Zwecke eingesetzt werden können. Insbesondere für medizinische Zwecke einzusetzende interpenetrierende Netzwerke der vorliegenden Erfindung können medizinische Wirkstoffe und Diagnostika, wie Kontrastmittel umfassen.
Die Materialien der vorliegenden Erfindung eignen sich insbesondere als Materialien auf dem medizinischen Gebiet, als Implantate, zur zielgesteuerten, stimuli-sensitiven Wirkstofffreisetzung, zur Bandaugmentation, als Bandscheibenersatz.

Die folgenden Anwendungsbeispiele erläutern die Erfindung.

### Herstellung interpenetrierender Netzwerke

Netzwerke wurden erhalten durch Bestrahlung von geschmolzenen Mischungen mit UV-Licht einer Wellenlänge von 308 nm. Die Mischungen umfassten jeweils ein Dimethacrylatpolycaprolacton (DMPC) (Mn = 10000 g/mol), erhalten durch ringöffnende Polykondensation von Caprolacton und anschließende Umsetzung der Endgruppen, so dass beide Enden mit Methacrylatgruppen versehen waren. Darüber hinaus enthielten die Mischungen jeweils Polyesterurethane (PU) unterschiedlicher Ausgestaltung (d.h. mit unterschiedlichem Gehalt an Pentadecalacton (PDL)), wie in der nachfolgenden Tabelle angegeben. Die Mischungen wurden erhalten durch Lösen der jeweiligen Komponenten in Chloroform, um eine Lösung mit einer Konzentration von 10 Gew.-% zu erhalten. Anschließend wurde diese Lösung in Hexanfraktion getropft, um die Ausgangsmaterialien in der erwünschten innigen Durchmischung auszufällen. Die Mischungen wurden bei 35°C bis zur Gewichtskonstanz getrocknet, dann bei 120°C geschmolzen. Die Polyesterurethane wurden erhalten durch ringöffnende Polymerisation von Caprolacton bzw. Pentadecalacton und Koppeln der erhaltenen Blöcke, die Diolendgruppen-funktionalisiert waren, durch Isocyanatverbindungen. Die Blöcke hatten jeweils ein Zahlenmittel des Molgewichts von 10000 g/mol.

Die Form in der die Vernetzung erfolgt entspricht der permanenten Form.

| Nr. | Gew.-% DMPC | Gew.-% PU | Mn PU (g/mol) | Gew.-% PDL im Netzwerk | Tg (°C) |
|---|---|---|---|---|---|
| 1 | 90 | 10 | 176000 | 6 | -62 |
| 2 | 80 | 20 | 176000 | 9 | -64 |
| 3 | 70 | 30 | 176000 | 13 | -69 |
| 4 | 60 | 40 | 176000 | 18 | -67 |
| 5+ | 50 | 50 | 176000 | | |
| 6+ | 70 | 30 | 170000 | 0 | -64 |
| 7 | 70 | 30 | 120000 | 5 | -61 |
| 8 | 70 | 30 | 196000 | 9 | -66 |
| 9 | 70 | 30 | 185000 | 22 | -69 |
| 10 | 70 | 30 | 192000 | 23 | |
| + Beispiel nicht erfindungsgemäß | | | | | |

Die polymeren interpenetrierenden Netzwerke wurden im Hinblick auf ihre weiteren thermischen und mechanischen Eigenschaften untersucht. Die Ergebnisse dieser Untersuchungen sind in der folgenden Tabelle zusammengefasst.

| Nr. | Tm1 (°C) | Tm2 (°C) | E-Modul bei 22°C (MPa) | Bruchdehnung bei 22°C (%) | Rückstellverhätnis nach 5 Zyklen (%)* |
|---|---|---|---|---|---|
| 1 | 55 | 89 | 45 | 250 | 99 |
| 2 | 52 | 90 | 52 | 310 | 96 |
| 3 | 51 | 89 | 53 | 390 | 83 |
| 4 | 54 | 92 | 65 | 375 | 87 |
| 6+ | 53 | | 56 | 425 | 98 |
| 7 | 53 | 88 | 53 | 425 | 97 |
| 8 | 53 | 89 | 62 | 445 | 93 |
| 9 | 53 | 92 | 70 | 375 | 87 |

| | | | | | |
|---|---|---|---|---|---|
| *thermischer Übergang bei Tm1 | | | | | |
| + Beispiel nicht erfindungsgemäß | | | | | |

Diese Experimente demonstrieren die überlegenen Eigenschaften der interpenetrierenden Netzwerke der vorliegenden Erfindung. Die Netzwerke zeichnen sich durch gute Werte für das die SMP-Eigenschaften kennzeichnende Gesamtrückstellverhältnis nach 5 Zyklen aus. Materialien des Stands der Technik zeigen hier häufig Werte von weniger als 80%. Figur 2 zeigt entsprechende Messungen für ein erfindungsgemäßes interpenetrierendes Netzwerk, wobei die Untersuchung im Hinblick auf den Form-Gedächtnis-Effekt bei Tm1 durchgeführt wurde.

Figur 3 zeigt entsprechende Daten für ein Experiment bei Tm2, wobei Rückstellverhältnisse von >99% erhalten werden.

Die in den bevorzugten Materialien vorliegende zweite, höhere Schmelztemperatur ermöglicht, dass die Materialien zwei Formen im "Gedächtnis" behalten. Durch die einfachen Grundbausteine der erfindungsgemäßen Netzwerke ist darüber hinaus eine gewisse Einfachheit der Synthese sichergestellt. Durch Variieren der Zusammensetzung, wie oben demonstriert, können gezielt polymere Materialien erhalten werden, die sich durch erwünschte Eigenschaftskombinationen auszeichnen. Figur 1 kann in diesem Zusammenhang noch einmal angeführt werden, da dort die beiden nacheinander ausgelösten Form-Gedächtnis-Effekte ersichtlich sind.

## Patentansprüche

1. Interpenetrierendes Netzwerk, erhältlich durch Vernetzen einer Polymerkomponente in der Gegenwart von Polyesterurethan, wobei das Polyesterurethan mindestens Segmente umfasst, abgeleitet von Pentadecalacton, wobei das Netzwerk eine kovalent vernetzte Polymerkomponente umfasst und das Polyesterurethan nicht kovalent vernetzt ist.

2. Interpenetrierendes Netzwerk nach Anspruch 1, wobei das Polyesterurethan Segmente umfasst, abgeleitet von Pentadecalacton und Caprolacton.

3. Interpenetrierendes Netzwerk nach Anspruch 1 oder 2, wobei die Polymerkomponente ein Polyester ist.

4. Interpenetrierendes Netzwerk nach Anspruch 1, 2 oder 3, wobei die Polymerkomponente mit Methacrylatgruppen an den Enden funktionalisiert ist.

5. Interpenetrierendes Netzwerk nach Anspruch 1, 2, 3 oder 4, wobei das Polyesterurethan Segmente aufweist, abgeleitet von Pentadecalacton, sowie Segmente, abgeleitet von teilkristallinen Segmenten, umfassend Polyestersegmente, Polyetherestersegmente und Polyethersegmente, wie Polycaprolactonsegmente (PCL), Polycaprolacton-co-polytetrahydrofuransegmente (PCL-co-pTHF), Polytetrahydrofuransegmente (pTHF). Polypropylenglycolsegmente (PPG) und Polyethylenglycolsegmente (PEG), sowie glasartigen Segmenten, umfassend Polyester und Copolyester, wie Poly-L-lactid-co-glycolid (ran) (PLGA) und Poly-DL-Lactid (P-DL-LA), bevorzugt PCL.

6. Interpenetrierendes Netzwerk nach Anspruch 1,2,3, 4 oder 5, wobei die Polymerkomponente ein Dimethacrylatderivat eines Polycaprolactons ist.

7. Verfahren zur Herstellung eines interpenetrierenden Netzwerks umfassend die Bestrahlung einer Schmelze, umfassend eine Polymerkomponente und Polyesterurethan, wie in Anspruch 1 definiert, mit UV-Licht.

8. Verfahren nach Anspruch 7, wobei die Polymerkomponente ein Polyester ist.

9. Verfahren nach Anspruch 7 oder 8, wobei die Polymerkomponente mit Methacrylatgruppen an den Enden funktionalisiert ist.

10. Verfahren nach Anspruch 7, 8 oder 9, wobei das Polyesterurethan Segmente aufweist, abgeleitet von Caprolacton und Pentadecalacton.

11. Verfahren nach Anspruch 7, 8, 9 oder 10, wobei die Polymerkomponente ein Dimethacralytderivat eines Polycaprolactons ist.

## Claims

1. Interpenetrating network, obtainable by crosslinking a polymer component in the presence of a polyester urethane, wherein the polyester urethane comprises at least segments derived from pentadecalactone, wherein the network comprises a covalently crosslinked polymer component and the polyester urethane is not covalently crosslinked.

2. Interpenetrating network in accordance with claim 1, wherein the polyester urethane comprises segments derived from pentadecalactone and caprolactone.

3. Interpenetrating network in accordance with claim 1 or 2, wherein the polymer component is a polyester.

4. Interpenetrating network in accordance with claim 1, 2 or 3, wherein the polymer component is terminally functionalised with methacrylate groups.

5. Interpenetrating network in accordance with claims 1, 2, 3 or 4, wherein the polyester urethane comprises segments derived from pentadecalactone, and segments derived from partially crystalline segments, comprising polyester segments, polyether ester segments and polyether segments, such as polycaprolactone segments (PCL), polycaprolactone-co-polytetrahydrofurane segments (PCL-co-pTHF), tetrahydrofurane segments (pTHF), polypropyleneglycol segments (PPG) and polyethyleneglycol segments (PEG), as well as glassy segments, comprising polyester and copolyester, such as poly-L-lactide-co-glycolide (ran) (PLGA) and poly-DL-lactide (P-DL-LA), preferably PCL.

6. Interpenetrating network in accordance with claims 1, 2, 3, 4 or 5, wherein the polymer component is a dimethacrylate of a polycaprolactone.

7. Process for preparing an interpenetrating network, comprising the irradiation of a melt, comprising a polymer component and a polyester urethane as defined in claim 1 with UV light.

8. Process in accordance with claim 7, wherein the polymer component is a polyester.

9. Process in accordance with claim 7 or 8, wherein the polymer component is terminally functionalised with methacrylate groups.

10. Process in accordance with claim 7, 8 or 9, wherein the polyester urethane comprises segments derived from caprolactone and pentadecalactone.

11. Process in accordance with claim 7, 8, 9 or 10, wherein the polymer component is a dimethacrylate of a polycaprolactone.

## Revendications

1. Réseau interpénétrant qui contient par réticulation un composant polymère en présence de polyesteruréthane, où le polyesteruréthane comprend au moins des segments, dérivés de pentadécalactone, où le réseau comprend un composant polymère réticulé covalent et le polyesteruréthane n'est pas réticulé pour être covalent.

2. Réseau interpénétrant selon la revendication 1, où le polyesteruréthane comprend des segments dérivés de pentadécalactone et de caprolactone.

3. Réseau interpénétrant selon la revendication 1 ou 2, où le composant polymère est un polyester.

4. Réseau interpénétrant selon la revendication 1, 2 ou 3, où le composant polymère est fonctionnalisé avec des groupes méthacrylate en bouts de chaînes.

5. Réseau interpénétrant selon la revendication 1, 2, 3 ou 4, où le polyesteruréthane présente des segments dérivés de pentadécalactone, ainsi que des segments, dérivés de segments partiellement cristallins, comprenant des segments de polyester, des segments de polyétherester et des segments de polyéther comme des segments de polycaprolactone (PCL), des segments de polycaprolactone-co-polytétrahydrofurane (PCL-co-pTHF), des segments de tétrahydrofurane (pTHF), des segments de polypropylèneglycol (PPG) et des segments de polyéthylèneglycol (PEG), ainsi que des segments vitreux, comprenant des polyesters et des copolyesters, comme le poly-L-acide lactique-co-glycolide(ran)(PLGA) et le poly-(DL)-acide lactique (P-DL-LA), le PCL étant préféré.

6. Réseau interpénétrant selon la revendication 1, 2, 3, 4 ou 5, où le composant polymère est un dérivé diméthacrylate d'une polycaprolactone.

7. Procédé de création d'un réseau interpénétrant qui comprend l'exposition à un rayonnement ultraviolet d'une fusion comprenant un composant polymère et le polyesteruréthane, selon la revendication 1.

8. Procédé selon la revendication 7, où le composant polymère est un polyester.

9. Procédé selon la revendication 7 ou 8, où le composant polymère est fonctionnalisé avec des groupes méthacrylate en bouts de chaînes.

10. Procédé selon la revendication 7, 8 ou 9, où le polyesteruréthane présente des segments dérivés de caprolactone et de pentadécalactone.

11. Procédé selon la revendication 7, 8, 9 ou 10, où le composant polymère est un dérivé diméthacrylate d'une polycaprolactone.
